Europäisches Patentamt

European Patent Office

Office européen des brevets

(19)

(11) Publication number: **0 227 110 B1**

(12) **EUROPEAN PATENT SPECIFICATION**

(45) Date of publication of patent specification: **11.12.91**    (51) Int. Cl.⁵: **C12N 15/13, C12P 21/02**

(21) Application number: **86117996.8**

(22) Date of filing: **23.12.86**

(54) **Human immunoglobulin G FC region protein and production thereof.**

(30) Priority: **26.12.85 JP 292298/85**

(43) Date of publication of application:
**01.07.87 Bulletin 87/27**

(45) Publication of the grant of the patent:
**11.12.91 Bulletin 91/50**

(84) Designated Contracting States:
**CH DE FR GB LI**

(56) References cited:
**EP-A- 0 125 023**
**EP-A- 0 147 283**
**EP-A- 0 184 187**

**PROC. NATL. ACAD. SCI. USA, vol. 79, March
1982, pages 1984-1988; J. ELLISON et al.:
"Linkage and sequence homology of two
human immunoglobulin gamma heavy chain
constant region genes"**

(73) Proprietor: **TEIJIN LIMITED**
**11 Minamihonmachi 1-chome Higashi-ku
Osaka-shi Osaka 541(JP)**

(72) Inventor: **Nakamura, Satoshi**
**Teijin Tama apart. 122 3-18-4, Tamadaira
Hino-shi Tokyo(JP)**
Inventor: **Masegi, Tsukio**
**Gurinhiru 103 1-8-11, Owada-cho
Hachioji-shi Tokyo(JP)**
Inventor: **Kitai, Kazuo**
**Teijin Musashino-ryo 3-5-18, Tamadaira
Hino-shi Tokyo(JP)**
Inventor: **Ichikawa, Yataro**
**2-11-7, Kotesashimachi
Tokorozawa-shi Saitama(JP)**

(74) Representative: **Vossius & Partner**
**Siebertstrasse 4 P.O. Box 86 07 67
W-8000 München 86(DE)**

## Description

BACKGROUND OF THE INVENTION

I. Field of the Invention

The present invention relates to a human immunoglobulin G Fc region protein, a DNA fragment coding for the protein, plasmids containing the DNA fragment, microorganisms transformed with the plasmid, and a process for production of the human immunoglobulin G Fc region protein using the transformed microorganism.

2. Description of the Related Art

An antibody is a protein present in the body fluid of vertebrates and capable of specific binding to an antigen. Proteins with a structure and functions related to the antibody proteins are generally designated as immunoglobulins. These immunoglobulins (abbreviated as Ig hereinafter) are classified to five classes, i.e., IgG, IgA, IgM, IgE, and IgD.

Among these immunoglobulins, IgG plays an important role in the biological defense against bacteria and viruses.

Human IgG comprises two heavy chains (abbreviated hereinafter as H chains) and two light chains (abbreviated hereinafter as L chains), wherein an H chain and an L chain are linked via a disulfide bond, and two H chains are linked via several disulfide bonds. When a human IgG molecule is subjected to the action of a protease such as papain, the molecule is cloven at approximately the center thereof into three fragments; two fragments having an antigen-binding activity (Fab region protein) and the other fragment having no antigen-binding activity but being easily crystallized (Fc region protein). The Fab region protein comprises an entire L chain and an amino terminal half of the H chain. The Fc region protein, on the other hand, comprises two fragments, each of which is a carboxy terminal half of the H chain, and these fragment are comprised of a hinge region, a $C_H2$ domain and a $C_H3$ domain, in that order, and links to the other fragment via several disulfide bonds at the hinge regions. The Fc region has a function as an effector.

As described above, since the IgG plays a important role in the biological defense, and is present in a large amount in blood, a human IgG rich $\gamma$-globulin fraction is obtained from human blood and used in an partially modified form as an immunological agent for administration into patients with symptoms of a serious nature. That is, the $\gamma$-globulin-containing pharmaceuticals are used as a supplement in hypogammaglobulinemia, and for the prevention and treatment of viral diseases. Recently, it has been suggested that the $\gamma$-globulin preparation is effective for the treatment of ideopathic chrombopenic purpura (ITP) (see, P. Imbach et al., Lancet, I, I228, I98I); and especially, it was suggested that the Fc region is important in the above-mentioned application (see, Boku et al., Rinsho Meneki, I5, 76 (I983). Moreover, immunocomplexes deposited on glomerulum in the case of systemic lupus erythematosus (SLE) were reported to be dissolved by adding human IgG Fc region protein (see, Kawasumi, Rinsho Meneki, I6, 240, I984).

Nevertheless, although the $\gamma$-globulin preparations and the Fc region protein derived therefrom are useful as the therapeutic agents for autoimmune diseases such as ITP and SLE, as mentioned above, such immunological preparations have certain disadvantages. For example, since the preparations are prepared from human blood which is difficult to obtain in a large amount as one lot, to obtain a substantial amount of the preparation, many lots of blood must be pooled. This gives rise to the problem of a diversity in the quality of the preparations, and a higher risk of viral contamination.

Therefore, it is much more desirable to produce the human immunoglobulin G Fc region protein by gene technology. The human IgG H chain is classified into four subclasses, i.e., $\gamma_1$ chain, $\gamma_2$ chain, $\gamma_3$ chain, and $\gamma_4$ chain. Among these subclasses, for the $\gamma_2$ and $\gamma_4$ chains, U. Krawinkel et al., EMBO J. L, 403 {I982) discloses a partial nucleotide sequence of a $\gamma_2$ chain gene and a $\gamma_4$ chain gene; and for the $\gamma_1$ chain, J. W. Ellison et al., Nucleic Acids Res. I0, 407I (1982) discloses a partial nucleotide sequence of a $\gamma_1$ chain gene. However, such reports do not disclose a DNA fragment capable of expressing a human IgG H chain gene.

SUMMARY OF THE INVENTION

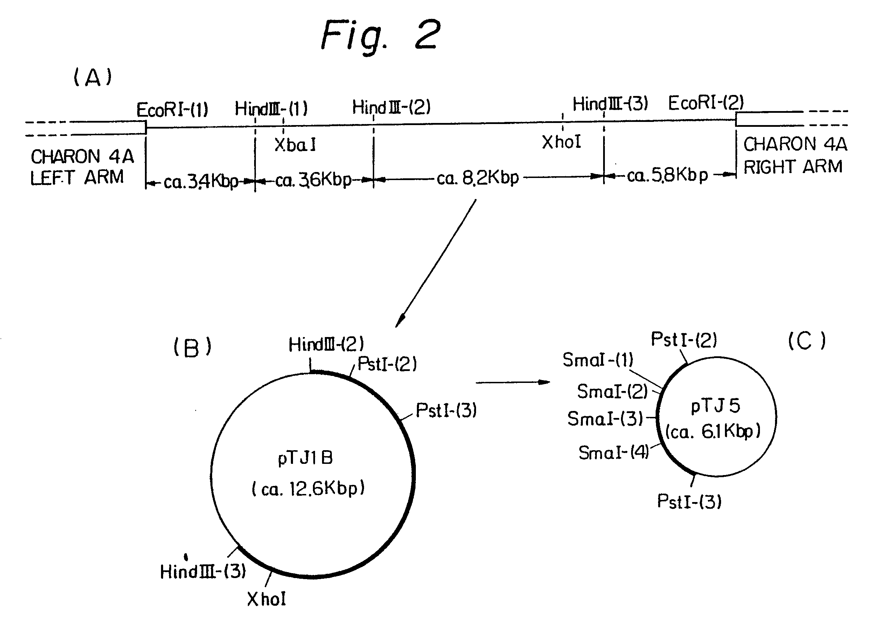

The present invention provides a monomeric human immunoglobulin G Fc region protein comprising an amino acid sequence from Thr at position 2 to Lys at position 224 in the amino acid sequence represented in Figure 2, essentially free of other proteins of human origin.

The present invention also provides a dimeric human immunoglobulin G Fc region protein derived from the above-mentioned monomeric proteins.

The present invention provides, a gene fragment coding for the above-mentioned monomeric protein; plasmids containing the above-mentioned gene fragment; and, microorganisms transformed with the above-mentioned plasmid.

Another object of the present invention is to provide a process for the production of the human immunoglobulin G Fc region protein by culturing the above-mentioned microorganism to accumulate the protein in the cultured product, and recovering the protein from the cultured product.

Other objects and advantages of the present invention will be apparent from the description set forth hereinbelow.

BRIEF DESCRIPTION OF THE DRAWINGS

Figures I-I and I-2 represent a part of the nucleotide sequence in an expression plasmid pFC362 for the human immunoglobulin G Fc region protein, and a corresponding amino acid sequence of the Fc region protein;

Fig. 2 represents a restriction endonuclease cleavage map of a clone containing the human immunoglobulin G gene, and a restriction endonuclease cleavage map of a subclone containing the Fc region gene;

Fig. 3 represents a process for the construction of a plasmid pFC70 containing a $C_H3$ part gene;

Fig. 4 represents a process for the construction of a plasmid pFC77 containing a $C_H2$-$C_H3$ part gene;

Fig. 5 represents a process for the construction of an expression plasmid pFC203 for the Fc region gene;

Fig. 6 represents a process for the construction of an expression plasmid pFC204 for the Fc region gene;

Fig. 7 represents a process for the construction of expression plasmids pFC203S and pFC203P for the Fc region gene;

Fig. 8 represents a process for the construction of an expression plasmid pFC2II for the Fc region gene;

Fig. 9 represents a process for the construction of expression plasmids pFC36I and pFC362 for the Fc region gene; and,

Fig. I0 represents a result of confirmation of the expression of the Fc region gene.

DESCRIPTION OF THE PREFERRED EMBODIMENT

The present invention is directed to the production of human immunoglobulin G Fc region protein in monomeric and dimeric forms. To this end, there are provided, a gene fragment coding for the above-mentioned Fc region protein; recombinant plasmids containing the gene fragment; recombinant microorganisms transformed with the plasmid; and a process for production of the Fc region protein using the recombinant microorganism.

In the present specification and drawings, amino acids, peptides, and nucleotide sequences, are described by using, optionally, abbreviations recommended by the IUPAC-IUB (Commission on Biological Nomenclature) or those conventionally used in the art.

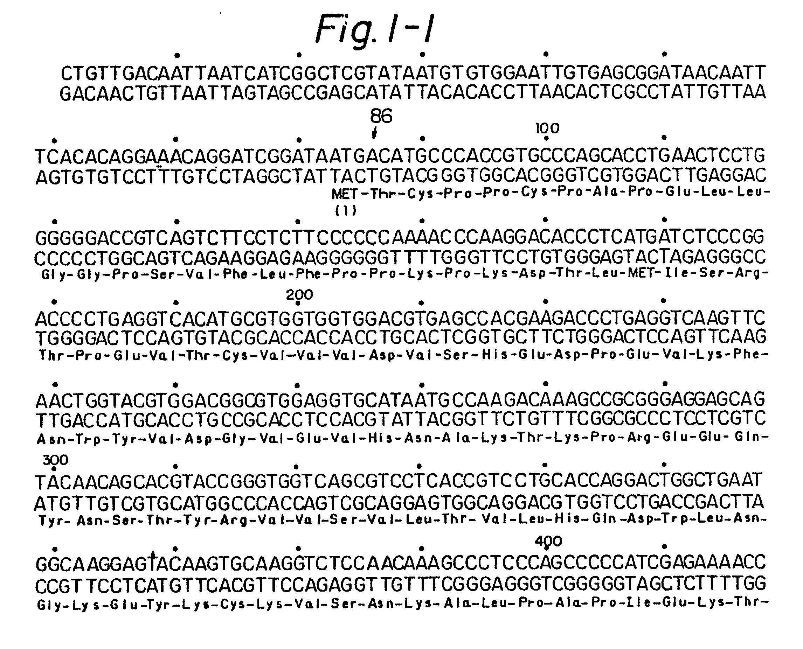

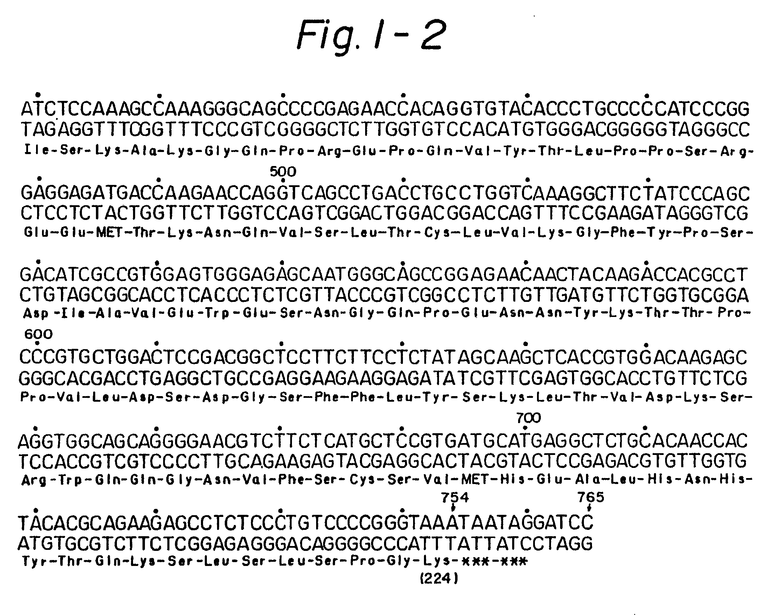

The monomeric human immunoglobulin G Fc region protein according to the present invention comprises an amino acid sequence from Thr at position 2 to Lys at position 224, as shown in Figures I-I and I-2, and essentially free of other proteins of human origin. In one embodiment, the present monomeric Fc region protein has an additional amino acid such as Met or Ser at position 1 in Figure 1-1. The protein may be glycosylated depending on the host used. Its glycosylation pattern may be different from the natural form.

The dimeric human immunoglobulin G Fc region protein may be derived from two molecules of the above-mentioned monomeric protein by natural or artificial association via disulfide bonds.

The gene fragment of the present invention encodes a polypeptide comprising amino acids from Thr at position 2 to Lys at position 224, as shown in the Figs. I-I and I-2. In one embodiment, the gene fragment is a double stranded DNA fragment which comprises a single, stranded coding DNA shown by a nucleotide sequence from A at a position 86 to A at a position 754 shown in the Figs. I-I and I-2, and a single stranded DNA complementary to the coding DNA.

In another embodiment, the gene fragment of the present invention encodes a polypeptide consisting of amino acids from Met at position I to Lys at position 224. In this embodiment, this gene fragment is a double stranded DNA fragment which consists of a single stranded coding DNA shown by a nucleotide sequence from A at position 83 to A at position 754 in the Figs. I-I and I-2, and a single stranded DNA complementary to the coding DNA.

The recombinant plasmid of the present invention may contain, in addition to the DNA fragment coding for the Fc region protein, an upstream start codon and one or more stop codons downstream of the DNA fragment coding for the Fc region protein. Moreover, the recombinant plasmid may contain a promotor region upstream of the start codon, for efficient expression of the human immunoglobulin G Fc region protein gene. Preferably, the promotor is a tryptophan operon promotor or a tac promotor. In this embodiment, this recombinant plasmid contains a double stranded DNA fragment which consists of a single stranded coding DNA shown by a nucleotide sequence from C at position I to G at position 765 in the Figs. I-I and I-2, and a single stranded DNA complementary to the coding DNA.

The process for preparation of a gene system including a gene fragment, recombinant plasmids, and transformed microorganisms, as well as the target protein, is summarized as follows:

(a) Preparation of human chromosomal DNA and gene library

Cells producing human IgG, for example, human lymphoma cell line ARH77 (see, K. H. Burk et al. J. Cancer Res. 38, 2508, I978), are cultured under appropriate conditions, for example, at a temperature of 37° C and at a carbon dioxide concentration of 5%, and the resulting cells are collected by centrifugation. The cells are lysed with a protease such as protease K in the presence of a detergent such as sodium dodecyl sulfate (SDS). The lysate is extracted by, for example, phenol, to eliminate proteins, and the human chromosomal DNA is obtained.

The chromosomal DNA thus obtained is then digested with a restriction enzyme such as Eco RI to obtain DNA fragments, which are then ligated to a phage vector such as Charon 4A vector (F.R. Blattner et al., Science, I96, I6I, I977), and the ligation products are subjected to in vitro packaging (A. Becker et al., Proc. Natl. Acad. Sci. USA, 72, 58I, I975) to obtain a human gene library. If a restriction enzyme other than Eco RI is used, or another phage vector with no Eco RI site as a cloning site is used, an appropriate linker DNA may be used to prepare the gene library.

(b) Preparation of restriction enzyme cleavage map of subclone

The phage in the gene library are transfected into a host such as E. coli LE 392 (ATCC 33572) to form plaques, which are then screened by plaque hybridization (W.P. Benton et al. Science, I96, I80, I977) to select the desired clones. The probe used for the screening is, for example, the human immunoglobulin H chain J region (which is a part of the Fab region and is present between a variable region having an antigen binding activity and a constant region having an effector function) gene labeled with $^{32}$P according to nick translation (P.W.J. Rigby et al. J. Mol. Biol. II3, 237, I977), or a synthetic oligonucleotide predicted to correspond to a part of an amino acid sequence of the human IgG Fc region protein, and labeled with $^{32}$P as above-mentioned.

For the DNA fragment of clones positive in the above-mentioned plaque hybridization, restriction enzyme cleavage site maps are prepared and DNA fragments derived from human chromosome are subcloned to a plasmid vector such as pBR322 (F. Bolivar et al. Gene, 2, 95, I977).

An insert DNA of the resulting subclones is sequenced according to the Maxam-Gilbert method (A.M. Maxam et al. Methods Enzymol. 65, 499, I980), or dideoxy chain termination method using MI3 phage (J. Messing et al. Nucleic Acids Res. 9, 309, I98I) and the like, to confirm the presence of the human IgG Fc region gene.

(c) Preparation of DNA fragment

Since the human IgG Fc region protein gene thus obtained is of human chromosomal origin, it contains introns and, therefore, cannot be expressed in microorganisms. Therefore, the Fc region gene is cloven with appropriate restriction enzymes to eliminate entirely the intron regions. If a part of the exon is deleted by the cleavage with the restriction enzymes, a chemically synthesized linker is used to repair the deleted part and link adjacent exons. Similarly, by using synthetic oligonucleotides, more than one translation stop codon in tandem (TGA, TAG or TAA) can be attached, in reading frame with the Fc region gene at the 3'-end thereof, to enhance the efficiency of its expression. The thus-obtained Fc region gene without any introns can be attached to a translation start codon upstream of and in reading frame with the gene. Moreover, the Fc region gene can be linked downstream to an appropriate promotor and a Shine-Dalgarno (S-D) sequence to form an expressible gene. Useful promotors include the tryptophan operon promotor (trp promotor), the lactose operon promotor (lac promotor), the tac promotor, the $P_L$ promotor, the $l_{PP}$ promotor, and the like. The trp promotor and the tac promotor are especially preferable. For efficient expression of the Fc region gene, the Fc region gene is preferably linked to a promotor, S-D sequence, and a translation start codon positioned upstream thereof, and to translation stop codons positioned downstream thereof. Most preferably, a promotor, an S-D sequence, a translation start codon, an Fc region gene, and translation stop codons are linked, in this order.

(d) Construction of recombinant plasmids and recombinant microorganisms

The expression plasmid can be constructed by inserting the expressible human IgG Fc region gene into an appropriate plasmid vector such as pBR322. The expression plasmids according to the present invention preferably include pFC203, pFC204, pFC203S, pFC203P, pFC2II, pFC36I, and pFC362.

Microbial hosts for expression of the human IgG Fc region gene include Escherichia coli, Bacillus subtilis and yeast, preferably E. coli. The above-mentioned plasmid can be incorporated into a microbial host such as E. coli according to a known method (M.V. Norgard et al. Gene 3, 279, I978).

The recombinant microorganisms thus obtained are cultured by a method known per se. The medium used for the culturing is, for example, an M9 medium (T. Maniatis et al. ed., Molecular Cloning, p 440, Cold Spring Harbor Laboratory, New York, I982) supplemented with glucose and casamino acids, to which medium is optionally added an antibiotic such as ampicillin to stabilize expression plasmids in the host cells.

The culturing is carried out under a condition suitable for the culturing of recombinant microorganisms, for example, at a temperature of 37°C for 2 to 36 hours with shaking, or aeration and agitation. If necessary, 3,β-indoleacrylic acid (when a trp promotor is used), or isopropyl-β,D-thiogalactoside (when a tac promotor is used) is added to the culture medium at the start of culturing, or during culturing, to induce the expression.

After the culturing, the resulting recombinant microbial cells are collected by a conventional means, such as centrifugation or filtration, the collected cells are suspended in an aqueous medium such as a phosphate buffer, the suspension is sonicated to disrupt the cells, and centrifuged to obtain a lysate. The amount of human IgG Fc region protein in the lysate can be determined by enzyme immunoassay using a commercially available rabbit antiserum containing the antihuman IgG Fc component and enzyme-labeled anti-rabbit Ig antibody.

The human IgG Fc region protein can be purified from the lysate according to a conventional process used for isolation and purification of a protein. Preferably, affinity column chromatography using anti-human IgG Fc component antibody is used.

The purified Fc region protein in monomeric form can be converted to a dimeric form wherein two monomeric proteins are linked via two disulfide bonds similar to natural immunoglobulin by, for example, a method disclosed by Chance et al. (R.E. Chance et al., Peptides: the Seventh U.S. Peptide Symposium Proceedings, D.H. Rich & E. Gross ed., 72I-728, Piece Chemical Co., Rockford, IL., I98I).

A dimeric protein can be converted to a monomeric protein by reducing the dimeric protein under the conventional conditions for reductive cleavage of a disulfide bond, for example, by adding dithiothreitol or 2-mercaptoethanol. A mixture of a monomeric protein and a dimeric protein can be separated into individual forms by any conventional separation procedure, such as gel filtration techniques.

Examples

The present invention will now be further illustrated by, but is by no means limited to, the following examples.

Example I. Isolation of human chromosomal DNA

$3 \times 10^8$ cells of the human lymphoma cell line ARH77 were disrupted with a glass rod, and the disrupted cells were treated with protease K (Sigma) in the presence of 2% SDS, added to phenol saturated with an aqueous solution containing I0 mM Tris-HCl (pH 8.0) and I mM EDTA, and mixed. The aqueous phase was separated from the phenol phase by centrifugation to obtain the aqueous phase (phenol extraction), which was then dialysed against an aqueous solution containing 20 mM Tris-HCl (pH 7.5), I00 mM NaCl, and 5 mM EDTA. The dialysate was treated with ribonuclease A (Sigma), again extracted with phenol, and dialysed against an aqueous solution containing I0 mM Tris-HCl (pH 8.0) and I mM EDTA to obtain about I.2 mg of human chromosomal DNA (N. Blin et al., Nucleic Acids Res. 3, 2303, I976).

Example 2. Preparation of human gene library

I50 μg of the human chromosomal DNA obtained in Example I was partially digested with restriction enzyme Eco RI (Takara Shuzo, Japan), and the digest was subjected to sucrose density gradient centrifugation in 10 to 40% (w/v) sucrose at 28,000 rpm (120,000 × g) for 15 hours at a temperature of 20°C, to obtain 4.3 μg of DNA fragments corresponding to I5 to 23 Kbp. 0.8 μg of the DNA fragments were then ligated with Charon 4A vector to obtain a hybrid DNA comprising the DNA fragments of human origin

EP 0 227 110 B1

franked with a right arm and a left arm of the Charon 4A vector. The ligation was carried out with T4 DNA ligase (Bethesda Research Laboratories) in an aqueous solution containing 66 mM Tris-HCl (pH 7.6), 6.6 mM MgCl₂ , I0 mM dithiothreitol and I mM ATP, at II°C for I2 hours. The hybrid DNA thus obtained was subjected to in vivo packaging to obtain a human gene library (I.8 × I0⁶ PFU/μg, containing 99% of the human chromosomal DNA).

Example 3. Screening for human immunoglobulin G gene

The gene library prepared in Example 2, comprising a population of the Charon 4A phage containing the DNA fragment of human origin, was used to transfect E. coli LE 392 to form plaques. Clones containing the human immunoglobulin gene were selected by a plaque hybridization method using ³²P-labeled human antibody H chain J gene as a probe. DNA containing the human immunoglobulin gene was prepared from the Charon 4A phage according to a method of Thomas et al. J. Mol. Biol. 9I, 3I5, 1974.

Example 4. Preparation of restriction map

I μg of Charon 4A DNA containing the human immunoglobulin gene was dissolved in 20 μl of a restriction enzyme buffer, 2 to 4 units of a restriction enzyme were added to the solution, and it was incubated for more than one hour. The incubation was carried out at 60°C for restriction enzymes Bst NI and Taq I, and at 37°C for other restriction enzymes. The restriction enzyme buffer was an aqueous solution containing 50 mM Tris-HCl (pH 7.4), I00 mM NaCl, and I0 mM MgSO₄ for Eco RI, Taq I, Xba I, and Xho I; an aqueous solution containing I0 mM Tris-HCl (pH 7.5), 60 mM NaCl, and 7 mM MgCl₂ for Bam HI, Cla I, Hind III, Pst I, Rsa I, and Sau 3AI; an aqueous solution containing I0 mM Tris-HCl (pH 7.4), 10 mM MgSO₄ , and I mM dithiothreitol for Bal I, Bst NI, Nae I, and Sst II; and an aqueous solution containing I0 mM Tris-HCl (pH 8.0), 20 mM KCl, 7 mM MgCl₂ , and 7 mM 2-mercaptoethanol for Sma I. Restriction enzymes Bst NI, Cla I and Nae I were obtained from New England Biolabs; Sst II was obtained from Bethesda Research Laboratories; Rsa I, Sau 3AI and Taq I were obtained from Nippon Gene; and other restriction enzymes were obtained from Takara Shuzo. Where two restriction enzymes were used, DNA was treated first with a restriction enzyme which acts at a lower salt concentration, and after the salt concentration in the reaction mixture was increased, treated with another restriction enzyme which acts at a higher salt concentration.

After the cleavage of DNA with the restriction enzyme, 4 μl of an aqueous solution of 0.25% bromophenol blue in 50% glycerol was added to the reaction mixture, and the mixture was subjected to agarose gel electrophoresis, wherein 0.8 to 2.5% of agarose concentration was used. This agarose was Type II for electrophoresis use obtained from Sigma. The electrophoresis buffer was an aqueous solution containing 40 mM Tris-CH₃COOH (pH 8.0) and I mM EDTA. Electrophoresis was carried out using a vertical gel 2 mm thick, at a voltage of 6 to 9 V/cm for I.5 to 3 hours. As a molecular weight marker, a cleavage product of λ phage DNA with Hind III (Boehringer Mannhein) was used. After the electrophoresis, DNA in the agarose gel was stained with 2 μg/ml of an ethidium bromide aqueous solution, and the agarose gel was irradiated with long wavelength UV to observe the restriction enzyme cleavage pattern of the DNA. By analyzing cleavage patterns obtained by each restriction enzyme digestion and by combinations of digestions with two restriction enzymes, the relative positions of restriction enzyme cleavage sites for some restriction enzymes were determined as shown in Fig. 2(A). Figure 2(A) represents a restriction map for human chromosomal DNA containing the human immunoglobulin G gene.

Example 5. Subcloning of human immunoglobulin G gene

3 μg of Charon 4A DNA containing the human immunoglobulin G gene was cloven with restriction enzyme Hind III according to the same procedure as used in Example 4, and the cleavage product was subjected to agarose gel electrophoresis (gel concentration 0.8%). A gel fragment containing a band corresponding to DNA of about 8.2 Kbp containing the human IgG Fc region gene was excised from the gel, and the excised gel fragment was dissolved in 3 volumes/weight of an 8 M NaClO₄ aqueous solution. A DNA fragment of about 8.2 Kbp in length was recovered from the agarose gel solution by a method disclosed by C.W. Chen et al, Anal. Biochem. I0I, 339 (I980).

On the other hand, I μg of plasmid pBR322 was cloven with restriction enzyme Hind III according to the procedure described in Example 4, and 0.5 units of alkaline phosphatase (E. coli C 75; Takara Shuzo) were added to the reaction mixture, and reacted at 45°C for one hour. After the reaction, the reaction mixture was extracted three times with phenol to inactivate and eliminate the alkaline phosphatase. This reaction

6

mixture containing pBR322 DNA treated with Hind III and alkaline phosphatase was mixed with the above-described Hind III fragment of 8.2 Kbp recovered from the agarose gel, the mixture then subjected to ethanol precipitation, and the precipitate dissolved in 50 $\mu$l of a ligation mixture (see Example 2). 2 units of T4 DNA ligase were added to the solution, and reacted for 12 hours at II°C to ligate the DNA fragments.

The ligation mixture was used to transform E. coli C 600 r⁻m⁻ (ATCC 33525) by a modified method of a conventional CaCl₂ method (M.V. Norgard). That is, E. coli C 600 r⁻m⁻ was cultured for 18 hours to prepare an inoculum, which was then inoculated to 5 $\mu$l of L medium (1% trypton, 0.5% of yeast extract, and 0.5% of NaCl, pH 7.2), and the E. coli was cultured to an extent corresponding to an optical density at 600 nm (OD$_{600}$) of 0.3. The cultured cells were washed two times in a cold magnesium buffer (0.1 M NaCl, 5 mM MgCl₂, 5 mM Tris-HCl, pH 7.6, 0°C), resuspended in a cold calcium buffer (100 mM CaCl₂, 250 mM KCl, 5 mM MgCl₂, 5 mM Tris-HCl, pH 7.6, 0°C), and the suspension was allowed to stand for 25 minutes at 0°C. The cells were separated by centrifugation and resuspended to a 1/10 volume of the calcium buffer to increase the cell concentration. The concentrated cell suspension was then mixed with the above-mentioned aqueous solution containing the ligated DNA at a volume ratio of 2:1. The mixture was maintained for 60 minutes at 0°C, and 1 ml of an LBG medium (1% trypton, 0.5% yeast extract was added, 1% NaCl and 0.08% glucose, pH 7.2), and culturing was carried out at 37°C for one hour with shaking. 100 $\mu$l/plate of the cultured broth was inoculated on a selective medium (L medium plate containing 30 $\mu$g/ml of ampicillin), and the plates were incubated at 37°C overnight to develop colonies of transformants. From the ampicillin resistant colonies, DNA was prepared by a conventional method, and analysed by agarose gel electrophoresis to confirm a desired subclone pTJIB (about 12.6 Kbp).

A restriction map of the subclone was prepared according to the procedure described in Example 4, and is shown in Fig. 2(B). As shown in Fig. 2(B), it was confirmed that three or four Pst I sites existed between Pst I(3) and Hind III(3). Note, one position was not confirmed.

Moreover, a Pst I(2) - Pst I(3) DNA fragment having a length of 1.7 Kbp was excised and inserted into a Pst I site of plasmid pBR322 to construct a plasmid pTJ5 (about 6.1 Kbp), according to the procedure as described for pTJIB. A desired clone was selected from the tetracycline resistant transformants, and a restriction map of the subclone thus obtained was as shown in Fig. 2(C).

## Example 6. Sequencing of DNA

A nucleotide sequence of the human immunoglobulin G Fc region gene was determined according to the Maxam-Gilbert method.

For example, about 50 $\mu$g of DNA of the subclone pTJ5 constructed as shown in Example 5 was cloven with Sma I according to the procedure described in Example 4, the resulting DNA fragments were dephosphorylated with an alkaline phosphatase, and labeled with [$\gamma$-³²P]-ATP using 5 units of polynucleotidekinase (P-L Biochemicals). The reaction using polynucleotidekinase was carried out in an aqueous solution containing 50 mM Tris-HCl (pH 9.5), 10 mM MgCl₂, and 5 mM dithiothreitol with 100 $\mu$Ci of [$\gamma$-³²P]-ATP (Amersham). The ³²P-labeled DNA fragments were cloven with Pst I, desired DNA fragments were separated by polyacrylamide gel electrophoresis (acrylamide concentration 5%), and extracted from the gel according to the procedure described hereinafter in Example 7. The ³²P-labeled Sma I-Pst I fragments thus obtained were subjected to a base-specific partial cleavage, and the product was separated by polyacrylamide gel electrophoresis (acrylamide concentration 8 to 23%) in 7 M urea. The gel was subjected to autoradiography at -80°C for 1 day, the cleavage pattern was analysed, and the result was used to determine a nucleotide sequence of the Fc region gene.

On the other hand, DNA of plasmid PTJ5 was cloven with Pst I, and the resulting DNA fragment was labeled with [$\alpha$-³²P]-ddATP using a 3'-terminal label kit (Amersham). The ³²P-labeled DNA fragments were cloven with Sma I, and desired DNA fragments were separated and purified by polyacrylamide gel electrophoresis (acrylamide concentration 5%). The ³²P-labeled Pst I-Sma I fragments were analysed as described above, and the result was used to determine a nucleotide sequence of the Fc region gene.

## Example 7. Construction of expression plasmid (cloning of C$_H$3 part gene)

DNA of plasmid pTJ5 constructed in Example 5 was cloven with a restriction enzyme Pst I according to the procedure described in Example 4, the resulting DNA fragments were separated by agarose gel electrophoresis (agarose concentration 0.8%), and a DNA fragment of about 1.7 Kbp containing the Fc region gene was recovered from the gel by the procedure described in Example 5. The DNA fragment thus obtained was cloven with a restriction enzyme Nae I by the procedure described in Example 4, and the resulting DNA fragments were separated by polyacrylamide gel electrophoresis (gel concentration 5%). A gel fragment containing a band corresponding to a DNA fragment of about 0.6 Kbp containing a $C_H3$ part gene was excised and disrupted, the disrupted gel particles were added with 2 to 5 ml of an elution buffer (500 mM ammonium acetate, I mM EDTA and 0.I% SDS, pH 7.5), and the mixture was shaken at $37°C$ overnight. The mixture was centrifuged to recover an aqueous phase containing the desired DNA fragment. The DNA fragment thus obtained was then cloven with restriction enzyme Rsa I by the procedure described in Example 4, the resulting DNA fragments separated by polyacrylamide gel electrophoresis (gel concentration 5%), and a DNA fragment of about 3I0 bp containing a $C_H3$ part gene was recovered from the gel as above mentioned.

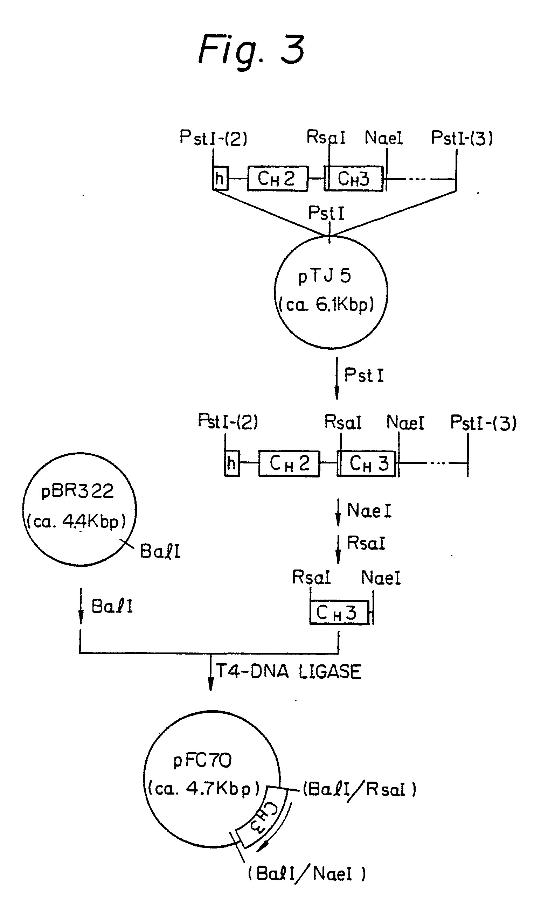

The Rsa I - Nae I DNA fragment containing the $C_H3$ part gene was inserted into the Bal I site of the plasmid pBR322 according to a procedure essentially similar to the procedure described in Example 5, to construct a plasmid pFC70 (about 4.7 Kbp) wherein the reading orientation of the $C_H3$ part gene was the same as the reading orientation of the tetracycline resistance gene in pBR322, i.e., clockwise orientation in Fig. 3). The process for constructing pFC70 is shown in Fig. 3.

Example 8. Construction of expression plasmid (ligation of $C_H2$ part gene and $C_H3$ part gene)

The DNA fragment of I.7 Kbp containing the Fc region gene obtained in Example 7 was cloven with restriction enzymes Sau 3AI and Taq I, the resulting DNA fragments were separated by polyacrylamide gel electrophoresis (acrylamide concentration 5%), and about 0.5 μg of DNA fragment having a length of about 240 bp containing the $C_H2$ part gene was recovered from the gel by the procedure described in Example 7.

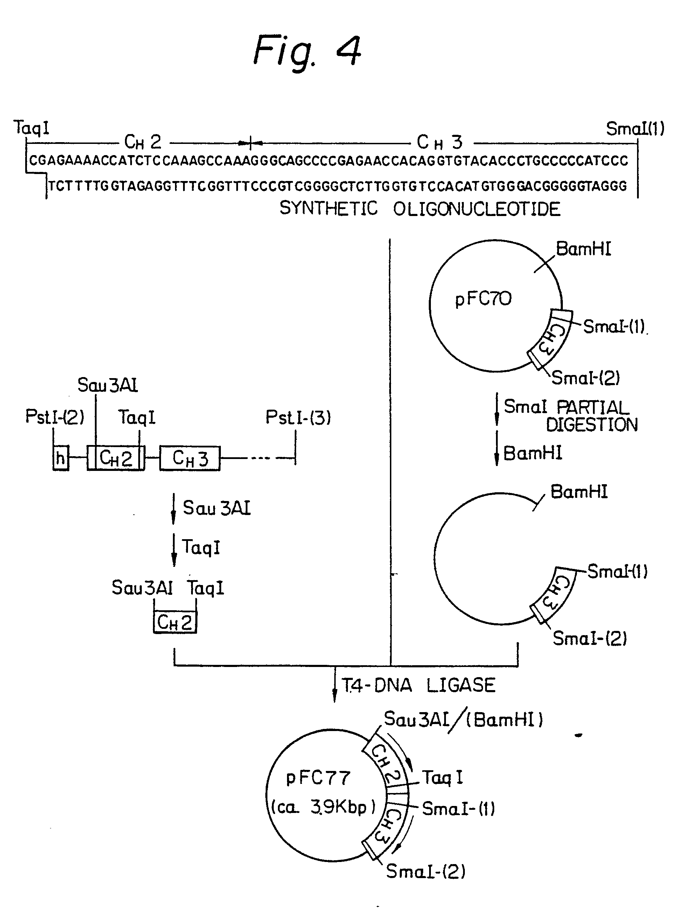

A double stranded oligonucleotide corresponding to a joint region between the $C_H2$ part gene and $C_H3$ part gene, having a nucleotide sequence shown in Fig. 4, was prepared by separately synthesizing a single stranded upper chain and a lower chain.

The single stranded oligonucleotides were synthesized by the phosphoamidite method using a fully-automatic DNA synthesizer (Applied Biosystems, Model 380A). The synthetic oligonucleotides were purified according to a manual disclosure by Applied Biosystems. That is, an aqueous ammonium solution containing synthetic oligonucleotides was maintained at $55°C$ overnight to deprotect the DNA base, and the solution was subjected to gel filtration using Sephadex G-50 fine gel (Pharmacia) to isolate a synthetic oligonucleotide with a high molecular weight. Next, the isolated product was subjected to polyacrylamide gel electrophoresis (acrylamide concentration 20%) in 7 M urea, and a migration pattern in the gel was observed to determine the position of a band corresponding to a desired oligonucleotide size. The desired part of the gel thus determined was then excised, and a desired synthetic oligonucleotide was recovered from the excised gel piece according to the procedure described in Example 7. Finally, the oligonucleotide solution was applied on a gel filtration column (Sephadex G-50) to obtain a purified synthetic oligonucleotide preparation. If necessary, the polyacrylamide gel electrophoresis was repeated to improve the purity of the oligonucleotide product. 0.I to I.0 μg of the purified synthetic oligonucleotide was treated with polynucleotide kinase in the presence of I mM ATP as described in Example 6, to phosphorylate the 5'-end of the oligonucleotide. Two phosphorylated synthetic oligonucleotides corresponding to the upper chain and lower chain were mixed, and the mixture was gradually cooled from $70°C$ to room temperature for annealing. As described above, a Taq I - Sma I DNA fragment of about 68 bp corresponding to a joint region between the $C_H2$ part gene and $C_H3$ part gene was obtained.

On the other hand, about 5 μg of DNA of the plasmid pFC70 was dissolved in the cleavage buffer for the restriction enzyme Sma I as described in Example 4, the solution was added with 2 to 5 units of Sma I, and incubated at $20°C$ for I5 to 45 minutes for partial digestion. The reaction mixture was extracted with phenol to inactivate the Sma I, and subjected to cleavage with Bam HI according to the procedure described in Example 4. The resulting DNA fragments were separated by agarose gel electrophoresis (agarose concentration 0.8%), and a Bam HI - Sma I(I) DNA fragment of about 3.6 Kbp containing most of the $C_H3$ part gene, as shown in Fig. 4, was recovered from the gel by the procedure described in Example 5.

The Sau 3AI - Taq I DNA fragment containing the $C_H2$ part gene; the Taq I - Sma I DNA fragment corresponding to the joint region between the $C_H2$ part gene and $C_H3$ part gene; and the Bam HI (Sau 3AI) - Sma I(I) DNA fragment containing the $C_H3$ part gene and a vector part, all prepared as above-mentioned, were mixed and ligated according to a procedure roughly the same as the procedure described in Example 5, to construct a plasmid pFC77 of about 3.9 Kbp wherein the $C_H2$ part gene and the $C_H3$ part gene were linked without an intervening intron, i.e., directly. The above-mentioned process for constructing the plasmid pFC77 is shown in Fig. 4.

Example 9. Construction of expression plasmid (ligation of Fc region gene and promotor)

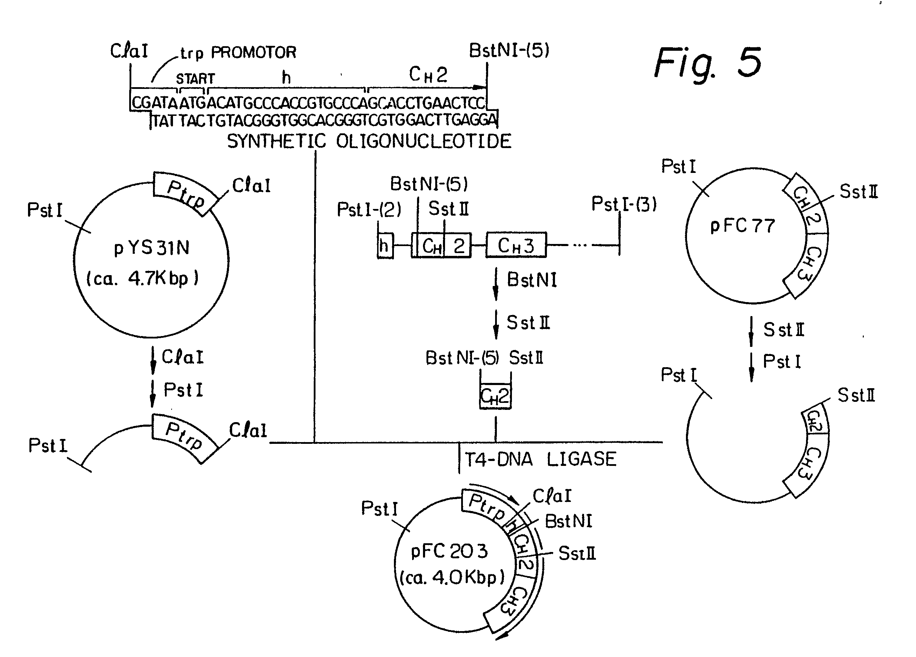

The DNA of the plasmid pFC77 obtained in Example 8 was cloven with restriction enzymes Sst II and Pst I by the same procedure described in Example 4, and after agarose gel electrophoresis (agarose concentration 0.8%), an Sst II - Pst I DNA fragment of about 2.7 Kbp containing the downstream part of the $C_H2$ part gene, the entire $C_H3$ part gene, and a part of the vector region, as shown in Fig. 5, was recovered from the gel by the prooedure described in Example 5.

On the other hand, the DNA fragment of about l.7 Kbp containing the Fc region gene obtained in Example 7 was cloven with restriction enzymes Bst NI and Sst II by the procedure described in Example 4, and after polyacrylamide gel electrophoresis (acrylamide concentration 5%), a Bst NI(5) - Sst II DNA fragment of about l7l bp containing an upstream part of the $C_H2$ part gene was recovered from the gel by the procedure described in Example 7.

Moreover, a double stranded oligonucleotide of about 39 bp corresponding to a joint region between a promotor and Fc region gene, shown in Fig. 5, was prepared by the procedure described in Example 8. This Cla I - Bst NI(5) DNA fragment contains a restriction enzyme Cla I site for ligation to a trp promotor, a translation start codon ATG, an h part gene, and an upstream part of the $C_H2$ part gene, in sequence in this order. Therefore, by using this DNA fragment, the Fc region (h-$C_H2$-$C_H3$ part) gene without any intron can be linked downstream of the tryptophan•operon•SD sequence within an appropriate distance.

On the other hand, the DNA of the plasmid pYS3lN of about 4.7 Kbp containing the trp promotor was cloven with restriction enzymes Pst I and Cla I by the procedure described in Example 4, and after agarose gel electrophoresis (agarose concentration 0.8%), a Pst I - Cla I DNA fragment of about l.l Kbp containing the trp promotor and a part of the vector region, as shown in Fig. 5, was recovered from the agarose gel by the procedure described in Example 5.

The Sst II - Pst I DNA fragment containing a downstream part of the $C_H2$ part gene, $C_H3$ part gene, and a part of the vector region; a Bst NI(5) - Sst II DNA fragment containing an upstream part of the $C_H2$ part gene; the Cla I - Bst NI(5) DNA fragment corresponding to the joint region between the promotor and the Fc region gene; and the Pst I - Cla I DNA fragment containing the trp promotor and a part of the vector region, all prepared as described above, were mixed and ligated to construct an expression plasmid pFC203 of about 4.0 Kbp for the Fc region (h-$C_H2$-$C_H3$ part) gene by a procedure roughly the same as the procedure described in Example 5. The above-mentioned process for construction of the pFC203 is shown in Fig. 5.

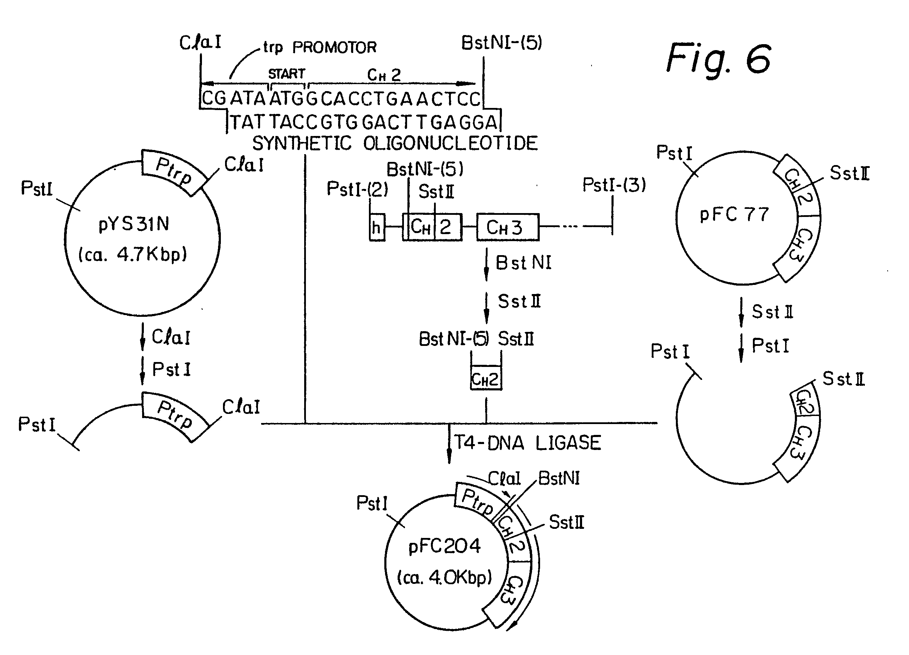

Moreover, a double stranded oligonucleotide having a nucleotide sequence shown in Fig. 6 corresponding to a joint region between the promotor and the $C_H2$ part gene was prepared and used to obtain an expression plasmid pFC204 for the Fc region ($C_H2$-$C_H3$ part) gene by essentially the same procedure as described above. The process for construction of pFC204 is shown in Fig. 6.

Example l0. Modification of expression plasmid (change in distance between the S-D sequence and translation start codon)

About 3 μg of DNA of the Fc region expression plasmid pFC203 obtained in Example 9 was cloven with restriction enzyme Cla I by the procedure described in Example 4, and dissolved in 40 μl of a polymerase buffer (50 mM Tris-HCl, pH 7.2, l0 mM $MgSO_4$ , 0.l mM DTT, and 50 mg/ml BSA), and 2 units of DNA polymerase I large fragment (Bethesda Research Laboratories) in the presence of 0.25 mM of dCTP and 0.25 mM of dGTP were added. The reaction mixture was incubated for 30 minutes at 37° C to produce blunt ends, and the reaction mixture was then extracted with phenol to inactivate the DNA polymerase I large fragment, and self-ligation was carried out by the procedure described in Example 2. The Fc region gene expression plasmid pFC203P was then constructed by the procedure described in Example 5. In this plasmid pFC203P, the distance between the S-D sequence and translation start codon is longer by 2 bp than that of pFC203.

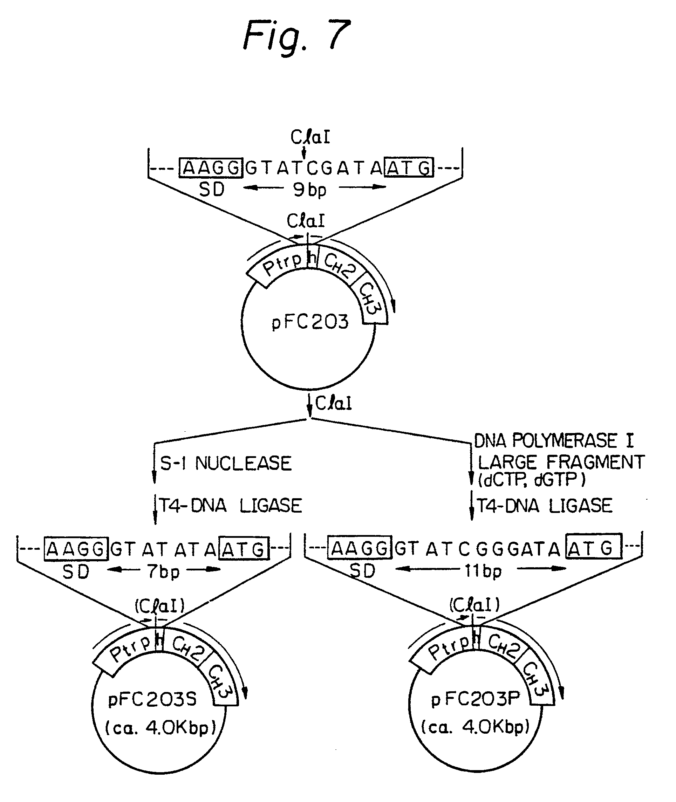

Figure 7 shows a process for the construction of pFC207P, and a nucleotide sequence between the S-D sequence and translation start codon ATG.

Next, about 3 μg of DNA of the Fc region gene expression plasmid pFC203 constructed in Example 9 was cloven with restriction enzyme Cla I by the procedure described in Example 4, and dissolved in 40 μl of an S-I nuclease buffer (30 mM sodium acetate, 50 mM NaCl, l mM $ZnSO_4$ and 5% glycerol, pH 4.6), and 25 units of S-I nuclease (Bethesda Research Laboratories) were added to the solution, and it was incubated at 37° C for 30 minutes to produce blunt ends. After the reaction mixture was extracted to inactivate the S-I nuclease, the Fc region gene expression plasmid pFC203S was constructed as described above. In this plasmid pFC203S, the distance between the S-D sequence and translation start codon was shorter by 2 bp than that of pFC203. Figure 7 also shows a process for the construction of pFC203S, and a nucleotide sequence between the S-D sequence and translation start codon ATG.

9

Example II. Modifioation of expression plasmid (incorporation of tandem stop codons)

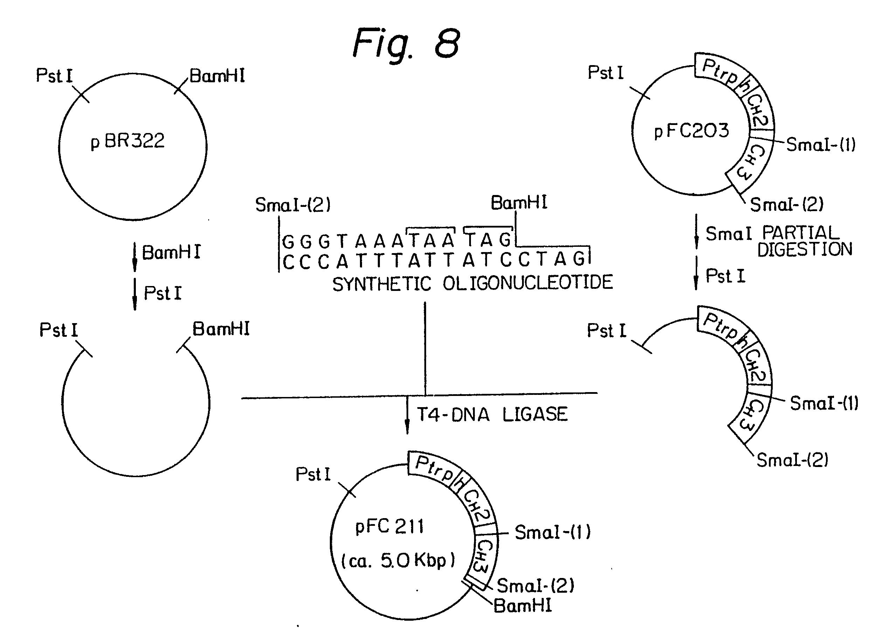

DNA of the Fc region gene expression plasmid pFC203 constructed in Example 9 was partially cloven with restriction enzyme Sma I, and then completely cloven with restriction enzyme Pst I by essentially the same procedure as described in Example 8. After agarose gel electrophoresis (agarose concentration 0.8%), an Sma I(2) - Pst I DNA fragment of about I.8 Kbp containing a major part of the Fc region gene and a part of the vector region, as shown in Fig. 8, was recovered from the agarose gel by same procedure described in Example 5.

On the other hand, a double stranded oligonucleotide of about I7 bp having a nucleotide sequence shown in Fig. 8 corresponding to a downstream part of the $C_H3$ part gene and translation stop codons was prepared by the procedure described in Example 8. This Sma I(2) - Bam HI DNA fragment contained a part of the $C_H3$ part gene, two tandem translation stop codons represented by the nucleotide sequence TAATAG, and a restriction enzyme Bam HI site for ligation to a vector. Therefore, this DNA fragment can be used to incorporate tandem translation stop codons into an expression plasmid.

The plasmid pBR322 DNA was cloven with restriction enzymes Pst I and Bam HI by the procedure described in Example 4, and after agarose gel electrophoresis (agarose concentration 0.8%), a Bam HI - Pst I DNA fragment of about 3.2 Kbp containing a major part of the vector region, as shown in Fig. 8, was recovered from the agarose gel by the procedure described in Example 5.

The Sma I(2) - Pst I DNA fragment containing a major part of the Fc region gene and a part of the vector region; the Sma I(2) - Bam HI DNA fragment containing a downstream part of the $C_H3$ part gene and tandem translation stop codons; and the Bam HI - Pst I DNA fragment containing a major part of the vector region, all prepared as described above, were mixed and ligated to construct the Fc region gene expression plasmid pFC2II of about 5.0 Kbp having tandem translation stop codons, by roughly the same procedure as described in Example 5. The above-mentioned process for the construction of pFC2II is shown in Fig. 8.

Example I2. Modification of expression plasmid (use of tac promotor)

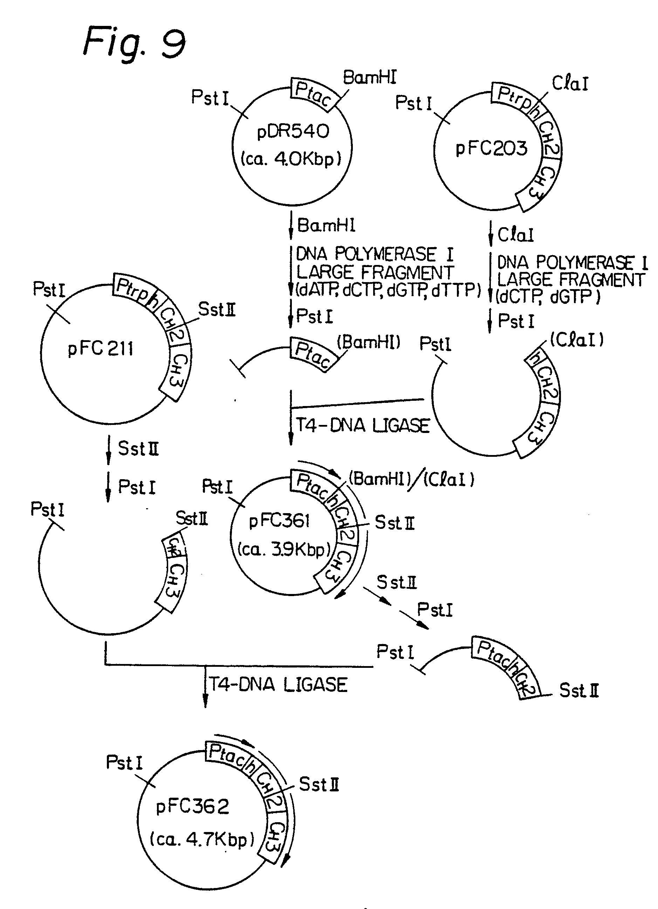

DNA of the Fc region gene expression plasmid pFC203 obtained in Example 9 was cloven with restriction enzyme Cla I by the procedure described in Example 4, and the resulting DNA was treated with DNA polymerase I large fragment in the presence of dCTP and dGTP to produce blunt ends by the procedure described in Example I0. Next, the blunt-ended DNA fragment was cloven with restriction enzyme Pst I by the procedure described in Example 4, and after agarose gel electrophoresis (agarose concentration 0.8%), a DNA fragment of about 2.8 Kbp containing an entire Fc region gene and a major part of a vector region, as shown in Fig. 9, was recovered.

On the other hand, DNA of the plasmid pDR540 of about 4.0 Kbp (Pharmacia) containing the tac promotor was cloven with restriction enzyme Bam HI by the procedure described in Example 4, and the resulting DNA was treated with DNA polymerase I large fragment in the presence of dGTP, dATP, dTTP, and dCTP to produce blunt ends, by the procedure described in Example I0. The blunt ended DNA fragment was then cloven with restriction enzyme Pst I by the procedure described in Example 4, and after agarose gel electrophoresis (agarose concentration 0.8%), a DNA fragment of about I.I Kbp containing the tac promotor and a part of the vector region, as shown in Fig. 9, was recovered from the agarose gel.

The DNA fragment of about 2.8 Kbp containing the entire Fc region gene and a major part of the vector region, and the DNA fragment of about I.I Kbp containing the tac promotor and a part of the vector region, both prepared as above, were mixed and ligated to construct the expression plasmid pFC36I of about 3.9 Kbp, wherein the Fc region gene was linked downstream to the tac promotor, by roughly the same method as described in Example 5. Figure 9 shows the above-mentioned process for the construction of pFC36I.

The DNA of the Fc region gene expression plasmid pFC36I thus obtained was cloven with restriction enzymes Sst II and Pst I by the procedure described in Example 4, and after agarose gel electrophoresis (gel concentration 0.8%), an Sst II - Pst I DNA fragment of about I.3 Kbp containing a part of the vector region, the tac promotor, and an upstream part of the Fc region gene, as shown in Fig. 4, was recovered from the agarose gel by the procedure described in Example 5.

On the other hand, the DNA of the Fc region gene expression plasmid pFC2II having tandem translation stop codons, obtained in Example II, was cloven with restriction enzymes Sst II and Pst I by the procedure described in Example 4, and then a Pst I - Sst II DNA fragment of about 3.6 Kbp containing a downstream part of the Fc region gene, tandem translation stop codons, and a major part of the vector region, as shown in Fig. 9, was obtained as described above.

The Sst II - Pst I DNA fragment containing a part of the vector region, the tac promotor, and an upstream part of the Fc region gene; and the Pst I - Sst II DNA fragment containing a downstream part of the Fc region gene, tandem translation stop codons, and a major part of the vector region, both prepared as above, were mixed and ligated to construct the expression plasmid pFC362 of about 4.9 Kbp wherein the tac promotor was followed by the Fc region gene with tandem translation stop codons, by roughly the same procedure as described in Example 5. Figure 9 shows the above-mentioned process for the construction of pFC362.

Figure 1 shows a part of the nucleotide sequence of the Fc region gene expression plasmid pFC362. In Fig. L, a polynucleotide represented by a nucleotide sequence from nucleotide position 86 to position nucleotide 754 encodes a polypeptide represented by an amino acid sequence from amino acid position 2 to amino acid position 224, i.e., human IgG Fc region protein.

Example I3. Expression of Fc region gene

E. coli C 600 r⁻m⁻ transformed with an Fc region gene expression plasmid constructed in Examples 9, I0, II, or I2 was cultured in M 9 medium (prepared by autoclaving an aqueous solution containing 0.6% $Na_2HPO_4$ , 0.3% $KH_2PO_4$ , 0.05% NaCl, and 0.1% $NH_4Cl$, pH 7.4, and then adding to this solution separately autoclaved aqueous solutions of $MgSO_4$ and $CaCl_2$ to a final concentration of 2 mM $MgSO_4$ and 0.1 mM $CaCl_2$) supplemented with 30 to 50 $\mu$g/ml ampicillin, 0.2% glucose, and 2 mg/ml casamino acids; or in L medium supplemented with 30 to 50 $\mu$g/ml ampicillin, at 37°C with shaking until the cell density reached $OD_{600}$ of 0.1. Subsequently, 3,$\beta$-indoleacrylic acid (Sigma) was added to the culture medium to a final concentration of 50 $\mu$g/ml when the trp promotor was used, or isopropyl-$\beta$,D-thiogalactoside (Sigma) to a final concentration of 5 mM when the tac promotor was used, and culturing was continued at 37°C with shaking until the cell density reached $OD_{600}$ of 0.5.

The cultured broth thus obtained was centrifuged to collect E. coli cells, and the cells were washed with a PBS buffer (20 mM phosphate buffer containing I50 mM NaCl, pH 7.4). The washed cells were then suspended in the PBS buffer, and the cells were disrupted using a sonicator (Kubota 200 M Type). The sonicated suspension was then centrifuged to obtain an E. coli lysate eliminating cell debris.

A Tris-HCl buffer (pH 6.8) was added to the E. coli lysate thus obtained to 60 mM, SDS to 2%, 2-mercaptoethanol to 4%, and glycerol to I0%, and the mixture was subjected to SDS-polyacrylamide gel electrophoresis (Suzuki, Iden, 31, 43, 1977). The acrylamide concentration of the separation gel was I2.5%, and the migration buffer was SDS-Tris-glycine buffer (U.K. Laemmli, Nature, 227, 680 (I970)). After the electrophoresis, proteins in the gel were electrophoretically transferred to and adsorbed on a nitrocellulose filter in a buffer containing 25 mM Tris, I92 mM glycine (pH 8.3) and 20% methanol (Western blotting).

The nitrocellulose filter on which proteins had been adsorbed was soaked in PBS buffer containing 5% bovine serum albumin for 60 minutes, and then the human immunoglobulin G Fc region protein was specifically stained by an indirect method using, as a primary antibody, a rabbit anti-human IgG-Fc component antiserum (Cappel) employing an Immun-blot assay kit (Bio Rad). The results are shown in Fig. I0.

In this confirmation procedure, a native human immunoglobulin G Fc region protein prepared by the procedure described hereinafter in the reference example was permitted to migrate on the same SDS-polyacrylamide gel parallel with the test samples, and the density of bands from test samples was compared with the density of the band of a known amount of the authentic (natural) sample to determine the amount of the former. The results for the various kinds of plasmids are set forth in the following table.

Table

| Plasmid | Medium | Amount of Fc produced (mg/l culture) |
|---------|--------|--------------------------------------|
| pFC204 | M9 | 1.0 ∿ 2.0 |
| pFC203 | M9 | 0.1 ∿ 0.2 |
| pFC211 | M9 | 0.1 ∿ 0.2 |
| pFC361 | L | 0.5 ∿ 0.8 |
| pFC362 | L | 0.8 ∿ 1.6 |

The E. coli C 600 r⁻m⁻ transformed with plasmid pFC362 produced 15 mg/l culture of the Fc region protein under an improved culture condition and medium composition. This amount corresponds to 10% of the E. coli cellular protein, and about 300,000 molecules per cell.

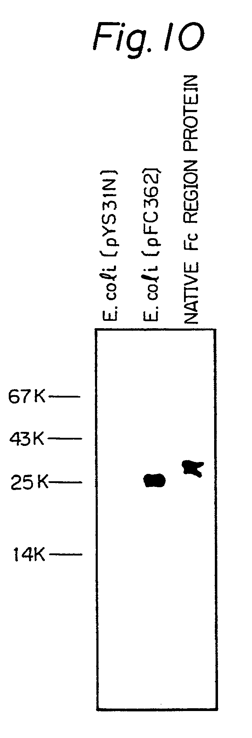

Since the molecular weight of Fc region protein produced by E. coli is lower by about 5000 dalton than that of the native Fc region protein, as seen from Fig. 10, the Fc region protein produced by E. coli is considered not to have carbohydrate chains.

Example 14. Purification of Fc region protein produced by E. coli

3 ml of an active type affinity support Affi-Gel 10 (Bio Rad) was coupled with 6.2 mg of affinity-purified sheep anti-human IgG-Fc component antibody (Cappel) in a 0.1 M MOPS buffer (pH 7.5, Nakarai Kagaku Yakuhin) to prepare an affinity column for purification of the E. coli-produced Fc region protein. The coupling was carried out at 4°C for two hours, and about 40% of the sheep anti-human IgG-Fc component antibody used was immobilized on the support.

The E. coli lysate prepared in Example 13 was passed through the affinity column prepared as described above specifically to adsorb the Fc region protein. The column was washed throughout with a PBS buffer and 20 mM phosphate buffer (pH 7.4) containing 500 mM NaCl, and then the Fc region protein was eluted with a 0.I.M glycine-HCl buffer (pH 2.3). The eluted Fc region protein was dialysed against water, and the dialysate was lyophilized. The lyophilizate was subjected to SDS polyacrylamine gel electrophoresis (acrylamide concentration I2.5%) by the procedure described in Example I2. After the electrophoresis, protein bands in the gel were stained with a silver stain reagent (Daiich Kagaku Yakuhin), and it was confirmed that a high purity E. coli-produced Fc region protein was obtained.

Reference Example Preparation of native human immunoglobulin G Fc region protein

0.3 g of human immunoglobulin G (Sigma), I7.5 mg of cysteine, and 7.2 mg of EDTA.2Na were dissolved in a PBS buffer (see Example I3), I50 μg of papain (Sigma Type IV) were added to the solution, and allowed to stand for 7 hours at 37°C. The papain-treated IgG was applied on a gel filtration column containing Sephadex G-200 super fine gel equilibrated with PBS buffer to separate the Fc region protein and Fab region protein, both generated by the papain treatment from intact IgG. An aqueous solution containing the Fc region protein and Fab region protein thus obtained was dialyzed against water, and the dialyzate was lyophilized. The lyophilizate was then applied to DE52.DEAE cellulose (Whatman) column for ion exchange chromatography. The column was washed with a I0 mM phosphate buffer (pH 7.4) to completely elute the Fab region protein, and then the Fc region protein was eluted by a linear elution gradient with from 0 mM to 350 mM NaCl in a I0 mM phosphate buffer (pH 7.4). Finally, dialyzation and lyophilization were carried out as described above to obtain a natural type human immunoglobulin G Fc region protein.

**Claims**

1. A monomeric human immunoglobulin G Fc region protein comprising the following amino acid sequence:

```
                              Thr-Cys-Pro-Pro-Cys-Pro-Ala-Pro-Glu-Leu-
                              (2)
     Leu-Gly-Gly-Pro-Ser-Val-Phe-Leu-Phe-Pro-Pro-Lys-Pro-Lys-
     Asp-Thr-Leu-MEt-Ile-Ser-Arg-Thr-Pro-Glu-Val-Thr-Cys-Val-
     Val-Val-Asp-Val-Ser-His-Glu-Asp-Pro-Glu-Val-Lys-Phe-Asn-
     Trp-Tyr-Val-Asp-Gly-Val-Glu-Val-His-Asn-Ala-Lys-Thr-Lys-
     Pro-Arg-Glu-Glu-Gln-Tyr-Asn-Ser-Thr-Tyr-Arg-Val-Val-Ser-
     Val-Leu-Thr-Val-Leu-His-Gln-Asp-Trp-Leu-Asn-Gly-Lys-Glu-
     Tyr-Lys-Cys-Lys-Val-Ser-Asn-Lys-Ala-Leu-Pro-Ala-Pro-Ile-
     Glu-Lys-Thr-Ile-Ser-Lys-Ala-Lys-Gly-Gln-Pro-Arg-Glu-Pro-
     Gln-Val-Tyr-Thr-Leu-Pro-Pro-Ser-Arg-Glu-Glu-MET-Thr-Lys-
     Asn-Gln-Val-Ser-Leu-Thr-Cys-Leu-Val-Lys-Gly-Phe-Tyr-Pro-
     Ser-Asp-Ile-Ala-Val-Glu-Trp-Glu-Ser-Asn-Gly-Gln-Pro-Glu-
     Asn-Asn-Tyr-Lys-Thr-Thr-Pro-Pro-Val-Leu-Asp-Ser-Asp-Gly-
     Ser-Phe-Phe-Leu-Tyr-Ser-Lys-Leu-Thr-Val-Asp-Lys-Ser-Arg-
     Trp-Gln-Gln-Gly-Asn-Val-Phe-Ser-Cys-Ser-Val-MET-His-Glu-
     Ala-Leu-His-Asn-His-Tyr-Thr-Gln-Lys-Ser-Leu-Ser-Leu-Ser-
     Pro-Gly-Lys , essentially free of other proteins of
                  (224)
     human origin.
```

2.  A protein according to claim 1 wherein said protein has an amino acid MET at an N-terminal thereof adjacent to Thr of amino acid position (2).

3.  A protein according to claim 1 wherein said protein is not glycosylated.

4.  A protein according to claim 1, produced by microbial cells.

5.  A dimeric human immunoglobulin G Fc region protein consisting of two monomeric protein chains of any one of claims 1 to 4.

6.  A gene fragment comprising a nucleotide sequence coding for the amino acid sequence of claim 1.

7.  A gene fragment according to claim 6 wherein the nucleotide sequence of a coding chain is as follows:

86
.
ACATGCCCACCGTGCCCAGCACCTGAACTCCTGGGGGGAC-
CGTCAGTCTTCCTCTTCCCCCCAAAACCCAAGGACACCCTCATGATCTCCCGGAC-
CCCTGAGGTCACATGCGTGGTGGTGGACGTGAGCCACGAAGACCCTGAGGTCAAG-
TTCAACTGGTACGTGGACGGCGTGGAGGTGCATAATGCCAAGACAAAGCCGCGGG-
AGGAGCAGTACAACAGCACGTACCGGGTGGTCAGCGTCCTCACCGTCCTGCACCA-
GGACTGGCTGAATGGCAAGGAGTACAAGTGCAAGGTCTCCAACAAAGCCCTCCCA-
GCCCCCATCGAGAAAACCATCTCCAAAGCCAAAGGGCAGCCCCGAGAACCACAGG-
TGTACACCCTGCCCCCATCCCGGGAGGAGATGACCAAGAACCAGGTCAGCCTGAC-
CTGCCTGGTCAAAGGCTTCTATCCCAGCGACATCGCCGTGGAGTGGGAGAGCAAT-
GGGCAGCCGGAGAACAACTACAAGACCACGCCTCCCGTGCTGGACTCCGACGGCT-
CCTTCTTCCTCTATAGCAAGCTCACCGTGGACAAGAGCAGGTGGCAGCAGGGGAA-
CGTCTTCTCATGCTCCGTGATGCATGAGGCTCTGCACAACCACTACACGCAGAAG-

754
AGCCTCTCCCTGTCCCCGGGTAAA

8. A gene fragment according to claim 7 wherein the gene fragment comprises the following additional nucleotide sequence upstream of and immediately adjacent to A of nucleotide position 86:

1
.
CTGTTGACAATTAATCATCGGCTCGTATAATGTGTGGAAT-
85
.
TGTGAGCGGATAACAATTTCACACAGGAAACAGGATCGGATAATG;

and the following additional nucleotide sequence downstream of and immediately adjacent to A of nucleotide position 754:

765
.
TAATAGGATCC

9. A plasmid containing a gene fragment according to claim 6.

10. A plasmid according to claim 9 wherein the plasmid is selected from the group consisting of plasmids pFC203 comprising about 4.0 kbp and having the restriction map shown in Fig. 5, pFC204 comprising about 4.0 kbp and having the restriction map shown in Fig. 6, pFC211 comprising about 5.0 kbp and having the restriction map shown in Fig. 8, pFC361 comprising about 3.9 kbp and having the restriction map shown in Fig. 9, and pFC362 comprising about 4.7 kbp and having the restriction map shown in Fig. 9.

11. A microorganism transformed with a plasmid according to claim 9.

12. A microorganism according to claim 11, wherein the microorganism is E. coli.

13. A process for production of a monomeric human immunoglobulin G Fc region protein or a dimer thereof, comprising culturing a microorganism according to claim 11, and recovering the monomeric or dimeric protein, or a mixture thereof, and optionally, separating the monomeric protein and dimeric protein, and optionally, converting the monomeric protein to the dimeric protein, and optionally, converting the dimeric protein to the monomeric protein.

## Revendications

1. Une protéine humaine monomérique de la région Fc d'immunoglobuline G comprenant la séquence d'acides aminés suivante:

```
        Thr-Cys-Pro-Pro-Cys-Pro-Ala-Pro-Glu-Leu-
            (2)
    Leu-Gly-Gly-Pro-Ser-Val-Phe-Leu-Phe-Pro-Pro-Lys-Pro-Lys-
    Asp-Thr-Leu-MEt-Ile-Ser-Arg-Thr-Pro-Glu-Val-Thr-Cys-Val-
    Val-Val-Asp-Val-Ser-His-Glu-Asp-Pro-Glu-Val-Lys-Phe-Asn-
    Trp-Tyr-Val-Asp-Gly-Val-Glu-Val-His-Asn-Ala-Lys-Thr-Lys-
    Pro-Arg-Glu-Glu-Gln-Tyr-Asn-Ser-Thr-Tyr-Arg-Val-Val-Ser-
    Val-Leu-Thr-Val-Leu-His-Gln-Asp-Trp-Leu-Asn-Gly-Lys-Glu-
    Tyr-Lys-Cys-Lys-Val-Ser-Asn-Lys-Ala-Leu-Pro-Ala-Pro-Ile-
    Glu-Lys-Thr-Ile-Ser-Lys-Ala-Lys-Gly-Gln-Pro-Arg-Glu-Pro-
    Gln-Val-Tyr-Thr-Leu-Pro-Pro-Ser-Arg-Glu-Glu-MET-Thr-Lys-
    Asn-Gln-Val-Ser-Leu-Thr-Cys-Leu-Val-Lys-Gly-Phe-Tyr-Pro-
    Ser-Asp-Ile-Ala-Val-Glu-Trp-Glu-Ser-Asn-Gly-Gln-Pro-Glu-
    Asn-Asn-Tyr-Lys-Thr-Thr-Pro-Pro-Val-Leu-Asp-Ser-Asp-Gly-
    Ser-Phe-Phe-Leu-Tyr-Ser-Lys-Leu-Thr-Val-Asp-Lys-Ser-Arg-
    Trp-Gln-Gln-Gly-Asn-Val-Phe-Ser-Cys-Ser-Val-MET-His-Glu-
    Ala-Leu-His-Asn-His-Tyr-Thr-Gln-Lys-Ser-Leu-Ser-Leu-Ser-
    Pro-Gly-Lys , essentiellement libre d'autres protéines
            (224)
    d'origine humaine.
```

2. Une protéine conforme à la revendication 1 où la dite protéine possède un acide aminé MET en position N-terminale, c'est à dire adjacent à la Thr (2) .

3. Une protéine conforme à la revendication 1 où la dite protéine n'est pas glycosylée.

4. Une protéine conforme à la revendication 1, produite par des cellules microbiennes.

5. Une protéine humaine dimérique de la région Fc d'immunoglobuline G constituée de deux chaines protéiques monomériques de l'une quelconque des revendications 1 à 4.

6. Un fragment de gène comprenant une séquence de nucléotides codant pour la séquence des acides aminés de la revendication 1.

7. Un fragment de gène conforme à la revendication 6 où la séquence de nucléotides d'une chaine codante est la suivante:

85

ACATGCCCACCGTGCCCAGCACCTGAACTCCTGGGGGGAC-

CGTCAGTCTTCCTCTTCCCCCCAAAACCCAAGGACACCCTCATGATCTCCCGGAC-

CCCTGAGGTCACATGCGTGGTGGTGGACGTGAGCCACGAAGACCCTGAGGTCAAG-

TTCAACTGGTACGTGGACGGCGTGGAGGTGCATAATGCCAAGACAAAGCCGCGGG-

AGGAGCAGTACAACAGCACGTACCGGGTGGTCAGCGTCCTCACCGTCCTGCACCA-

GGACTGGCTGAATGGCAAGGAGTACAAGTGCAAGGTCTCCAACAAAGCCCTCCCA-

GCCCCCATCGAGAAAACCATCTCCAAAGCCAAAGGGCAGCCCCGAGAACCACAGG-

TGTACACCCTGCCCCCATCCCGGGAGGAGATGACCAAGAACCAGGTCAGCCTGAC-

CTGCCTGGTCAAAGGCTTCTATCCCAGCGACATCGCCGTGGAGTGGGAGAGCAAT-

GGGCAGCCGGAGAACAACTACAAGACCACGCCTCCCGTGCTGGACTCCGACGGCT-

CCTTCTTCCTCTATAGCAAGCTCACCGTGGACAAGAGCAGGTGGCAGCAGGGGAA-

CGTCTTCTCATGCTCCGTGATGCATGAGGCTCTGCACAACCACTACACGCAGAAG-

754

AGCCTCTCCCTGTCCCCGGGTAAA

8. 8. Un fragment de gène conforme à la revendication 7 où le fragment de gène comprend la séquence de nucléotides additionnelle suivante en amont de et immédiatement adjacente au A de la position nucléotidique 86 :

1

CTGTTGACAATTAATCATCGGCTCGTATAATGTGTGGAAT-

85

TGTGAGCGGATAACAATTTCACACAGGAAACAGGATCGGATAATG;

et la séquence de nucléotides additionnelle suivante en aval de et immédiatement adjacente au A de la position nucléotidique 754 :

765

TAATAGGATCC

9. Un plasmide contenant un fragment de gène conforme à la revendication 6.

10. Un plasmide conforme à la revendication 9 où le plasmide est sélectionné à partir du groupe constitué des plasmides pFC203 comprenant environ 4,0 kpb et possédant la carte de restriction représentée sur la Fig.5, pFC204 comprenant environ 4,0 kpb et possédant la carte de restriction représentée sur la Fig.6, pFC211 comprenant environ 5,0 kpb et possédant la carte de restriction représentée sur la Fig.8, pFC361 comprenant environ 3,9 kpb et possédant la carte de restriction représentée sur la Fig.9, et pFC362 comprenant environ 4,7 kpb et possédant la carte de restriction représentée sur la Fig.9.

11. Un micro-organisme transformé avec un plasmide conforme à la revendication 9.

**12.** Un micro-organisme conforme à la revendication 11, où le micro-organisme est E.Coli.

**13.** Un procédé pour la production d'une protéine humaine monomérique de la région Fc d'immunoglobuline G ou un dimère de celle-ci, comprenant les étapes consistant à faire la culture d'un micro-organisme conforme à la revendication 11, et à récupérer la protéine monomérique ou dimérique, ou un mélange de celles-ci, et facultativement, à séparer la protéine monomérique et la protéine dimérique, et facultativement, à convertir la protéine monomérique en la protéine dimérique, et facultativement, à convertir la protéine dimérique en la protéine monomérique.

**Patentansprüche**

**1.** Monomeres menschliches Immunglobulin G Fc-Region-Protein enthaltend die nachfolgende Aminosäuresequenz:

```
        Thr-Cys-Pro-Pro-Cys-Pro-Ala-Pro-Glu-Leu-
             (2)
        Leu-Gly-Gly-Pro-Ser-Val-Phe-Leu-Phe-Pro-Pro-Lys-Pro-Lys-
        Asp-Thr-Leu-MEt-Ile-Ser-Arg-Thr-Pro-Glu-Val-Thr-Cys-Val-
        Val-Val-Asp-Val-Ser-His-Glu-Asp-Pro-Glu-Val-Lys-Phe-Asn-
        Trp-Tyr-Val-Asp-Gly-Val-Glu-Val-His-Asn-Ala-Lys-Thr-Lys-
        Pro-Arg-Glu-Glu-Gln-Tyr-Asn-Ser-Thr-Tyr-Arg-Val-Val-Ser-
        Val-Leu-Thr-Val-Leu-His-Gln-Asp-Trp-Leu-Asn-Gly-Lys-Glu-
        Tyr-Lys-Cys-Lys-Val-Ser-Asn-Lys-Ala-Leu-Pro-Ala-Pro-Ile-
        Glu-Lys-Thr-Ile-Ser-Lys-Ala-Lys-Gly-Gln-Pro-Arg-Glu-Pro-
        Gln-Val-Tyr-Thr-Leu-Pro-Pro-Ser-Arg-Glu-Glu-MET-Thr-Lys-
        Asn-Gln-Val-Ser-Leu-Thr-Cys-Leu-Val-Lys-Gly-Phe-Tyr-Pro-
        Ser-Asp-Ile-Ala-Val-Glu-Trp-Glu-Ser-Asn-Gly-Gln-Pro-Glu-
        Asn-Asn-Tyr-Lys-Thr-Thr-Pro-Pro-Val-Leu-Asp-Ser-Asp-Gly-
        Ser-Phe-Phe-Leu-Tyr-Ser-Lys-Leu-Thr-Val-Asp-Lys-Ser-Arg-
        Trp-Gln-Gln-Gly-Asn-Val-Phe-Ser-Cys-Ser-Val-MET-His-Glu-
        Ala-Leu-His-Asn-His-Tyr-Thr-Gln-Lys-Ser-Leu-Ser-Leu-Ser-
        Pro-Gly-Lys ,
             (224)
```

im wesentlichen frei von anderen Proteinen menschlichen Ursprungs.

**2.** Protein nach Anspruch 1, wobei das Protein eine Aminosäure MET am N-Terminus davon angrenzend an Thr in Aminosäureposition (2) aufweist.

**3.** Protein nach Anspruch 1, wobei das Protein nicht glykosiliert ist.

**4.** Protein nach Anspruch 1, hergestellt durch mikrobiologische Zellen.

**5.** Dimeres menschliches Immunglobulin G Fc-Region-Protein bestehend aus zwei monomeren Proteinketten nach einem der Ansprüche 1 bis 4.

**6.** Genfragment enthaltend eine Nukleotidsequenz, die die Aminosäuresequenz nach Anspruch 1 codiert.

17

7. Genfragment nach Anspruch 6, wobei eine codierende Kette die nachfolgende Nucleotidsequenz aufweist:

86
ACATGCCCACCGTGCCCAGCACCTGAACTCCTGGGGGGAC-
CGTCAGTCTTCCTCTTCCCCCCAAAACCCAAGGACACCCTCATGATCTCCCGGAC-
CCCTGAGGTCACATGCGTGGTGGTGGACGTGAGCCACGAAGACCCTGAGGTCAAG-
TTCAACTGGTACGTGGACGGCGTGGAGGTGCATAATGCCAAGACAAAGCCGCGGG-
AGGAGCAGTACAACAGCACGTACCGGGTGGTCAGCGTCCTCACCGTCCTGCACCA-
GGACTGGCTGAATGGCAAGGAGTACAAGTGCAAGGTCTCCAACAAAGCCCTCCCA-
GCCCCCATCGAGAAAACCATCTCCAAAGCCAAAGGGCAGCCCCGAGAACCACAGG-
TGTACACCCTGCCCCCATCCCGGGAGGAGATGACCAAGAACCAGGTCAGCCTGAC-
CTGCCTGGTCAAAGGCTTCTATCCCAGCGACATCGCCGTGGAGTGGGAGAGCAAT-
GGGCAGCCGGAGAACAACTACAAGACCACGCCTCCCGTGCTGGACTCCGACGGCT-
CCTTCTTCCTCTATAGCAAGCTCACCGTGGACAAGAGCAGGTGGCAGCAGGGGAA-
CGTCTTCTCATGCTCCGTGATGCATGAGGCTCTGCACAACCACTACACGCAGAAG-
754
AGCCTCTCCCTGTCCCCGGGTAAA

8. Genfragment nach Anspruch 7, wobei das Genfragment die nachfolgende zusätzliche Nucleotidsequenz stromaufwärts vom und unmittelbar angrenzend an das A in Nucleotidposition 86 umfaßt:

1
CTGTTGACAATTAATCATCGGCTCGTATAATGTGTGGAAT-
85
TGTGAGCGGATAACAATTTCACACAGGAAACAGGATCGGATAATG;

und die nachfolgende zusätzliche Nukleotidsequenz stromabwärts vom und unmittelbar angrenzend an das A in Nukleotidposition 754:

765
TAATAGGATCC

9. Plasmid enthaltend ein Genfragment nach Anspruch 6.

10. Plasmid nach Anspruch 9, wobei das Plasmid ausgewählt ist aus der Gruppe bestehend aus den Plasmiden pFC203, das etwa 4,0 kbp umfaßt und die in Figur 5 dargestellte Restriktionskarte aufweist, pFC204, das etwa 4,0 kbp umfaßt und die in Figur 6 dargestellte Restriktionskarte aufweist, pFC211, das etwa 5,0 kbp umfaßt und die in Figur 8 dargestellte Restriktionskarte aufweist, pFC361, das etwa 3,9 kbp umfaßt und die in Figur 9 dargestellte Restriktionskarte aufweist und pFC362, das etwa 4,7 kbp umfaßt und die in Figur 9 dargestellte Restriktionskarte aufweist.

**11.** Mikroorganismus transformiert mit einem Plasmid nach Anspruch 9.

**12.** Mikroorganismus nach Anspruch 11, wobei der Mikroorganismus E. coli ist.

**13.** Verfahren zur Herstellung eines monomeren menschlichen Immunglobulin G Fc-Region-Proteins oder eines Dimers davon, das die Züchtung eines Mikroorganismus nach Anspruch 11 und die Gewinnung des monomeren oder dimeren Proteins oder eines Gemisches davon umfaßt und gegebenenfalls die Trennung des monomeren und des dimeren Proteins und gegebenenfalls die Umwandlung des monomeren in das dimere Protein und gegebenenfalls die Umwandlung des dimeren in das monomere Protein.

## Fig. I-I

CTGTTGACAATTAATCATCGGCTCGTATAATGTGTGGAATTGTGAGCGGATAACAATT
GACAACTGTTAATTAGTAGCCGAGCATATTACACACCTTAACACTCGCCTATTGTTAA

86
100

TCACACAGGAAACAGGATCGGATAATGACATGCCCACCGTGCCCAGCACCTGAACTCCTG
AGTGTGTCCTTTGTCCTAGGCTATTACTGTACGGGTGGCACGGGTCGTGGACTTGAGGAC
MET-Thr-Cys-Pro-Pro-Cys-Pro-Ala-Pro-Glu-Leu-Leu-
(1)

GGGGGACCGTCAGTCTTCCTCTTCCCCCCCAAAACCCAAGGACACCCTCATGATCTCCCGG
CCCCCTGGCAGTCAGAAGGAGAAGGGGGGGTTTTGGGTTCCTGTGGGAGTACTAGAGGGCC
Gly-Gly-Pro-Ser-Val-Phe-Leu-Phe-Pro-Pro-Lys-Pro-Lys-Asp-Thr-Leu-MET-Ile-Ser-Arg-

200
ACCCCTGAGGTCACATGCGTGGTGGTGGACGTGAGCCACGAAGACCCTGAGGTCAAGTTC
TGGGGACTCCAGTGTACGCACCACCACCTGCACTCGGTGCTTCTGGGACTCCAGTTCAAG
Thr-Pro-Glu-Val-Thr-Cys-Val-Val-Val-Asp-Val-Ser-His-Glu-Asp-Pro-Glu-Val-Lys-Phe-

AACTGGTACGTGGACGGCGTGGAGGTGCATAATGCCAAGACAAAGCCGCGGGAGGAGCAG
TTGACCATGCACCTGCCGCACCTCCACGTATTACGGTTCTGTTTCGGCGCCCTCCTCGTC
Asn-Trp-Tyr-Val-Asp-Gly-Val-Glu-Val-His-Asn-Ala-Lys-Thr-Lys-Pro-Arg-Glu-Glu-Gln-

300
TACAACAGCACGTACCGGGTGGTCAGCGTCCTCACCGTCCTGCACCAGGACTGGCTGAAT
ATGTTGTCGTGCATGGCCCACCAGTCGCAGGAGTGGCAGGACGTGGTCCTGACCGACTTA
Tyr-Asn-Ser-Thr-Tyr-Arg-Val-Val-Ser-Val-Leu-Thr-Val-Leu-His-Gln-Asp-Trp-Leu-Asn-

400
GGCAAGGAGTACAAGTGCAAGGTCTCCAACAAAGCCCTCCCAGCCCCCATCGAGAAAACC
CCGTTCCTCATGTTCACGTTCCAGAGGTTGTTTCGGGAGGGTCGGGGGTAGCTCTTTTGG
Gly-Lys-Glu-Tyr-Lys-Cys-Lys-Val-Ser-Asn-Lys-Ala-Leu-Pro-Ala-Pro-Ile-Glu-Lys-Thr-

# Fig. I - 2

```
ATCTCCAAAGCCAAAGGGCAGCCCCGAGAACCACAGGTGTACACCCTGCCCCCATCCCGG
TAGAGGTTTCGGTTTCCCGTCGGGGCTCTTGGTGTCCACATGTGGGACGGGGGTAGGGCC
Ile-Ser-Lys-Ala-Lys-Gly-Gln-Pro-Arg-Glu-Pro-Gln-Val-Tyr-Thr-Leu-Pro-Pro-Ser-Arg-
                                   500
GAGGAGATGACCAAGAACCAGGTCAGCCTGACCTGCCTGGTCAAAGGCTTCTATCCCAGC
CTCCTCTACTGGTTCTTGGTCCAGTCGGACTGGACGGACCAGTTTCCGAAGATAGGGTCG
Glu-Glu-MET-Thr-Lys-Asn-Gln-Val-Ser-Leu-Thr-Cys-Leu-Val-Lys-Gly-Phe-Tyr-Pro-Ser-

GACATCGCCGTGGAGTGGGAGAGCAATGGGCAGCCGGAGAACAACTACAAGACCACGCCT
CTGTAGCGGCACCTCACCCTCTCGTTACCCGTCGGCCTCTTGTTGATGTTCTGGTGCGGA
Asp-Ile-Ala-Val-Glu-Trp-Glu-Ser-Asn-Gly-Gln-Pro-Glu-Asn-Asn-Tyr-Lys-Thr-Thr-Pro-
600
CCCGTGCTGGACTCCGACGGCTCCTTCTTCCTCTATAGCAAGCTCACCGTGGACAAGAGC
GGGCACGACCTGAGGCTGCCGAGGAAGAAGGAGATATCGTTCGAGTGGCACCTGTTCTCG
Pro-Val-Leu-Asp-Ser-Asp-Gly-Ser-Phe-Phe-Leu-Tyr-Ser-Lys-Leu-Thr-Val-Asp-Lys-Ser-
                                                         700
AGGTGGCAGCAGGGGAACGTCTTCTCATGCTCCGTGATGCATGAGGCTCTGCACAACCAC
TCCACCGTCGTCCCCTTGCAGAAGAGTACGAGGCACTACGTACTCCGAGACGTGTTGGTG
Arg-Trp-Gln-Gln-Gly-Asn-Val-Phe-Ser-Cys-Ser-Val-MET-His-Glu-Ala-Leu-His-Asn-His-
                                               754        765
TACACGCAGAAGAGCCTCTCCCTGTCCCCGGGTAAATAATAGGATCC
ATGTGCGTCTTCTCGGAGAGGGACAGGGGCCCATTTATTATCCTAGG
Tyr-Thr-Gln-Lys-Ser-Leu-Ser-Leu-Ser-Pro-Gly-Lys-***-***
                        (224)
```

EP 0 227 110 B1

# Fig. 2

(A)

(B)

(C)

EP 0 227 110 B1

## Fig. 3

EP 0 227 110 B1

# Fig. 4

Fig. 5

Fig. 6

# Fig. 7

*Fig. 8*

# Fig. 9

Fig. 10